# EUROPEAN PATENT SPECIFICATION

(11) **EP 3 875 081 B1**
(45) Date of publication and mention of the grant of the patent: **09.07.2025**
(21) Application number: 19878899.4
(22) Date of filing: 28.10.2019
(51) Int. Cl.: A61K 9/20, A61K 9/16, A61K 31/165, A61P 35/00, A61K 9/19

(54) **PHARMACEUTICAL COMPOSITION CONTAINING ALKYL CARBAMOYL NAPHTHALENYLOXY OCTENOYL HYDROXYAMIDE PHOSPHATE, TARTRATE OR COMBINATION THEREOF, AND PREPARATION METHOD THEREFOR**
PHARMAZEUTISCHE ZUSAMMENSETZUNG MIT ALKYLCARBAMOYL-NAPHTHALENYLOXYOCTENOYL-HYDROXYAMIDPHOSPHAT, TARTRAT ODER EINE KOMBINATION DAVON UND VERFAHREN ZU IHRER HERSTELLUNG
COMPOSITION PHARMACEUTIQUE CONTENANT DU PHOSPHATE D'ALKYL CARBAMYL NAPHTALÉNYLOXY OCTÉNYL HYDROXYAMIDE, DU TARTRATE OU UNE COMBINAISON DE CEUX-CI, ET SON PROCÉDÉ DE PRÉPARATION

(30) Priority: 29.10.2018 KR 20180129576
(43) Date of publication of application: 08.09.2021
(73) Proprietor: Crystalgenomics, Inc., Seongnam-si, Gyeonggi-do 13488 (KR)
(72) Inventor: CHO, Jae Pyoung, Gunpo-si Gyeonggi-do 15822 (KR); CHO, Joong Myung, Seoul 05553 (KR)
(74) Representative: Schön, Christoph
(86) International application number: PCT/KR2019/014234
(87) International publication number: WO 2020/091326

(56) References cited:
- WO-A1-2007/052938
- WO-A1-2008/054154
- KR-A- 20070 101 284
- KR-A- 20180 136 741
- KR-B1- 100 696 139
- KR-B1- 100 711 944
- KR-B1- 100 814 092
- KR-B1- 101 388 596
- HWANG, J. J ET AL: "A novel histone deacetylase inhibitor, CG0006, induces cell death through both extrinsic and intrinsic apoptotic pathways", ANTI-CANCER DRUGS, vol. 20, no. 9, 2009, pages 815 - 821, XP009517492, ISSN: 0959-4973, DOI: 10.1097/CAD.0b013e3283300a0f

## Description

### Technical Field

The present invention relates to a pharmaceutical composition comprising an alkylcarbamoyl naphthalenyloxy octenoyl hydroxyamide phosphate compound, an alkylcarbamoyl naphthalenyloxy octenoyl hydroxyamide tartrate compound or a combination thereof.

In addition, the present invention relates to a method for preparing the pharmaceutical composition.

### Background Art

A histone is a basic protein that binds to DNA in the eukaryotic cell nucleus, and reversible histone acetylation occurs on the amino group of a specific lysine residue in each molecule of the histone. The histone acetylation reaction is related to the formation of high-order structure of chromatin or the cell-division cycle, so it is involved in the regulation of expression of gene information, and stably regulated by histone acetyltransferases (HATs) and histone deacetylases (HDACs). It is known that these enzymes neutralize the positive charge of lysine residues (4 residues in the case of H4) present at the amino terminal of the histone by acetylation to induce transcriptional activity or release electric charge again by deacetylation to inhibit transcription, thereby inducing an equilibrium of the level of histone acetylation to regulate gene expression at the level of transcription.

HDAC has recently been found to play a role in promoting cell proliferation as it is highly expressed in poor environmental conditions such as hypoxia, low glucose and cell carcinogenesis to inhibit the expression of cell proliferation. Therefore, it is recognized as an important factor in regulating cell carcinogenesis and differentiation. That is, while high acetylation of chromatin inhibits cell proliferation and promotes differentiation, HDAC plays a crucial role in inducing cell proliferation through deacetylation of histones. This is supported by the results of inhibition of cell proliferation or angiogenesis by treatment with HDAC inhibitors. There is a need for the development of HDAC inhibitors having higher selectivity and excellent medicinal efficacy. Accordingly, the present inventors confirmed the possibility of alkylcarbamoyl naphthalenyloxy octenoyl hydroxyamide as an HDAC inhibitor, and thus have been conducting research on this.

However, since the alkylcarbamoyl naphthalenyloxy octenoyl hydroxyamide has a property of absorbing moisture in the atmosphere, there may occur a problem that is vulnerable to physicochemical stability. A number of purification operations for removal of related substances generated by moisture absorption can increase the production cost, and high hygroscopicity makes difficult to maintain the solid state, causing difficulties in mass-producing solid preparations. There is a disadvantage in that a separate freezing storage device or means such as packaging is required.

Therefore, there is a need for a study on a pharmaceutical composition as a stabilized HDAC inhibitor, which has rapid expression of effects and is convenient to manufacture.
WO 2007/052938 A1 discloses an alkylcarbamoyl naphthalenyloxy octenoylhydroxyamide derivative for inhibiting the enzyme activity of histone deacetylase, a method for preparing same and a pharmaceutical composition comprising same.
WO 2008/054154 A1 discloses naphthalenyloxypropenyl derivatives for inhibiting the enzyme activity of histone deacetylase.
KR 100 814 092 B1 discloses an alkylcarbamoyl naphthalenyloxy octenoyl hydroxyamide derivative, its pharmaceutically acceptable salt, a method for preparing the derivative, and a pharmaceutical composition containing the derivative or the salt to inhibit the enzymatic activity of histone deacetylase.

### Detailed Description of the Invention

### Technical Problem

The present invention provides a pharmaceutical composition in the form of a tablet, granule, powder, capsule, dry syrup or injection comprising an alkylcarbamoyl naphthalenyloxy octenoyl hydroxyamide hydrochloride compound, an alkyl carbamoyl naphthalenyloxy octenoyl hydroxyamide tartrate compound or a combination thereof.

In addition, it provides a method for preparing the pharmaceutical composition.

### Solution to Problem

In order to solve the above problems, the present invention provides a pharmaceutical composition as defined in appended claim 1.

Preferred embodiments are defined in appended claims 2 to 4.

According to another aspect, the present invention provides a method for preparing a pharmaceutical composition as defined in appended claim 5.

Preferred embodiments are defined in appended claims 6 to 10.

### Effect of the Invention

According to the pharmaceutical composition as defined in appended claim 1, it is possible to improve stability against moisture while maintaining properties such as medicinal efficacy and effective amount. In addition, by improving hygroscopicity, production and commercialization processes of the preparations can be simplified. In particular, by maintaining stability during the manufacturing process to prevent the production of related substances, it is possible to provide a pharmaceutical composition having safety and easy of manufacture.

### Brief Description of Drawings

Fig. 1 is a graph showing the dissolution pattern of tablets comprising alkylcarbamoyl naphthalenyloxy octenoyl hydroxyamide phosphate.
Fig. 2 is a graph showing the dissolution pattern of capsules comprising alkylcarbamoyl naphthalenyloxy octenoyl hydroxyamide phosphate.
Fig. 3 is a graph showing the results of measuring the amount of related substances produced in Example 12.
Fig. 4 is a graph showing a change in moisture content.
Fig. 5 is a graph comparing the amount of related substances produced in Examples and Comparative Examples.
Fig. 6 is a graph showing the amount of related substances produced in Example 13 according to storage temperature.
Fig. 7 is a graph showing the amount of related substances produced in Example 14 according to storage temperature.
Fig. 8 is a photograph showing appearance of the composition for injection having a lyophilized form comprising alkylcarbamoyl naphthalenyloxy octenoyl hydroxyamide phosphate.

### Best Mode for Carrying out the Invention

In the following description, detailed description of known functions will be omitted if it is determined that it may obscure the gist of the present invention.

The term "pharmaceutical composition", as used herein may be described interchangeably with "pharmacological composition" and "pharmaceutically acceptable composition" and refers to any composition which can be a relatively non-toxic to a subject to be administered and have harmless effective action. In addition, it may refer to any organic or inorganic compound formulation in that side effects resulting from the composition do not impair the efficacy of the drug, and that does not cause serious irritation to a subject to be administered by the compound and does not impair the biological activities and properties of the compound.

As used herein, the term 'subject to be administered' may be used interchangeably with 'subject to be applied', 'individual to be administered' and 'organism to be administered', and may refer to any animals including humans in need of HDAC inhibition.

Hereinafter, the pharmaceutical composition as defined in appended claim 1 will be described in more detail.

The possibility of alkylcarbamoyl naphthalenyloxy octenoyl hydroxyamide or a derivative thereof as a histone deacetylase (HDAC) inhibitor has been confirmed (Korean Patent No. 0814092). In order to improve stability against moisture while maintaining properties such as medicinal efficacy and effective amount of alkylcarbamoyl naphthalenyloxy octenoyl hydroxyamide or its derivatives, the present inventors have studied the most physicochemically ideal and stable form of the salt among various types of acceptable salts.

In particular, the present invention provides a pharmaceutical composition comprising a compound of formula 1 (alkylcarbamoyl naphthalenyloxy octenoyl hydroxyamide phosphate), a compound of formula 2 (alkyl carbamoyl naphthalenyloxy octenoyl hydroxyamide tartrate) or a combination thereof in the form of a tablet, granule, powder, capsule, dry syrup or formulated for injection, 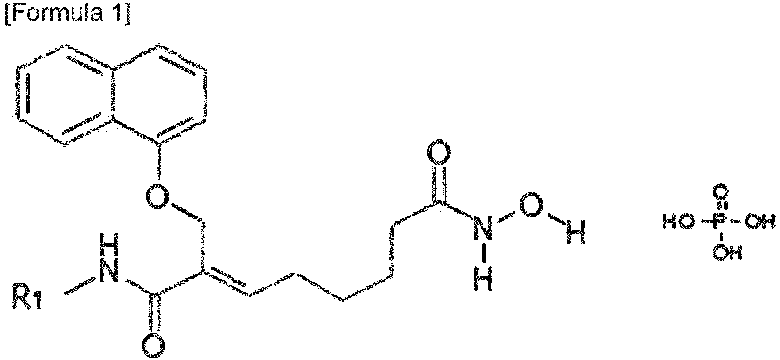 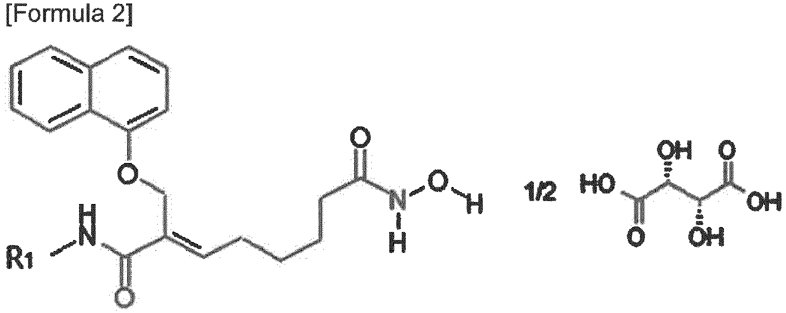 wherein in formula 1 or 2,
R₁ is N,N-dimethylpropylamine.

According to another embodiment of the present invention, there is provided a method for preparing a pharmaceutical composition comprising a compound of formula 1, a compound of formula 2, or a combination thereof, comprising:
1) forming a free base solution of (E)-N1-(3-(dimethylamino)propyl)-N8-hydroxy-2-((naphthalen-1-yloxy)methyl)-2-octenediamide in a first organic solvent, and
2) adding phosphoric acid or tartaric acid to the free base solution, wherein the pharmaceutical composition is in the form of a tablet, granule, powder, capsule, dry syrup or formulated injection.

According to an embodiment, the first organic solvent may comprise one or more selected from the group consisting of methanol, ethanol, propanol, tetrahydrofuran, chloroform, N,N-dimethylformamide (DMF), dimethyl sulfoxide (DMSO), acetonitrile and ethyl acetate. For example, it may comprise one or more selected from the group consisting of methanol, ethanol, propanol, tetrahydrofuran, chloroform, N,N-dimethylformamide (DMF) and dimethyl sulfoxide (DMSO), which have relatively high solubility.

According to an embodiment, the method may further comprise additionally adding a second solvent having a lower solubility than the first organic solvent of step 1). For example, the solvent having a lower solubility than the first organic solvent of step 1) may comprise one or more selected from the group consisting of alcohols including methanol, ethanol and propanol, tetrahydrofuran, acetonitrile and acetone. For example, after adding an organic solvent to alkylcarbamoyl naphthalenyloxy octenoyl hydroxyamide derivative as defined in appended claim 1, the precipitation, that is, the formation of a salt is observed, and if necessary, a solvent with a lower solubility than the added organic solvent may be additionally added to observe the precipitation. The addition of the solvent having a lower solubility may be repeated 2 to 5 times, for example, 2 times to obtain a salt.

The pharmaceutical composition of the present invention is provided in the form of a tablet, granule, powder, capsule, dry syrup or formulated for injection. In addition, it may be provided in the form of oral administration or injection.

The pharmaceutical composition for oral administration may comprise at least one diluent selected from the group consisting of microcrystalline cellulose, mannitol, lactose and lactose, at least one lubricant selected from the group consisting of talc, magnesium stearate, sodium stearyl fumarate and glyceryl behenate, and at least one binder selected from the group consisting of polyvinylpyrrolidone, hydroxypropyl methylcellulose and hydroxypropyl cellulose. In addition, for example, the coating layer may be included in an amount of 1 to 10% by weight based on 100% by weight of a tablet or capsule. Specifically, for example, the coating layer may include a water-soluble coating base material, and a commonly used coating base material may be used. More specifically, for example, it includes a coating base material including polyvinyl alcohol derivatives, methacrylic acid derivatives and polyacrylic acid derivatives, and for example, one or two or more selected from the group consisting of Opadry^{®}, Kollicoat^{®}, and hydroxypropyl methylcellulose (HPMC) may be used, for example polyvinyl alcohol containing Opadry^{®} having a relatively excellent effect of blocking moisture and light may be used.

When preparing a granule composition for oral administration, it is preferable not to use purified water as a binding solvent in consideration of the property of unstable moisture stability. Ethanol may be used that can be easily removed during the manufacturing process. Magnesium stearate, which is a lubricant used in a conventional composition for oral administration, may not be suitable for mixing with the alkylcarbamoyl naphthalenyloxy octenoyl hydroxyamide according to the present invention, so alternatives can be used.

According to an embodiment, in addition to the additives as described above, a pharmaceutical excipient having excellent compatibility with the compound can be added.

The injection composition can be provided in a liquid form because the alkylcarbamoyl naphthalenyloxy octenoyl hydroxyamide component is a water-soluble material that is well soluble in water. The use of solubilizing agents and other additives generally used to improve solubility of poorly soluble substances are not essential. It is preferable to use a minimum amount of additive as it may have unstable compatibility with the additives. More specifically, it can be prepared by dissolving in nitrogen-purged water for injection and then lyophilizing.

According to an embodiment, the method may further comprise sterilizing. The sterilization method may include dry heat sterilization, pressurized or reduced pressure sterilization, filter sterilization, gas sterilization, or radiation sterilization. In the filter sterilization method, a nitrocellulose membrane filter may be used, for example, and the filter having 0.45 µm or 0.2 µm pore size may be used. The method may further comprise sterilizing by a high-temperature decompression sterilization method or aseptic filtration method.

According to another embodiment of the present invention, it is possible to provide a pharmaceutical composition for an anticancer agent containing the compound of formula 1, the compound of formula 2, or a combination thereof as defined in appended claim 1 as an effective ingredient.

Hereinafter, embodiments of the present invention will be described in detail so that those skilled in the art can easily carry out the present invention.

### Examples 1 to 4

In order to prepare a tablet composition having convenience of dosing and easy of manufacture, a tablet composition was prepared using alkylcarbamoyl naphthalenyloxy octenoyl hydroxyamide phosphate and additives having established stability in the compositions as shown in Table 1, based on the results of compatibility studies.

(E)-N1-(3-(dimethylamino)propyl)-N8-hydroxy-2-((naphthalen-1-yloxy)methyl)-2-octenediamide phosphate compound (CG200745PPA) was used as alkylcarbamoyl naphthalenyloxy octenoyl hydroxyamide phosphate. The weight in the table below is in mg.

Owing to the nature of poor flowability of CG200745PPA, a tablet composition was prepared by a granulation method using ethanol as a binding solvent, instead of a direct compression method. In addition, due to the characteristics of CG200745PPA raw materials, it can be difficult to separate the tablet and the punch when subjected to the tableting pressure in the tableting process. Therefore, the ratio of the excipient added was increased, thereby minimizing the influence of appearance of the main component.

In addition, in Table 2, according to the degree to which the composition is jammed in the machine when punching the tablet, the mold release is expressed as good and poor. According to the degree of sticking to the machine surface, the sticking is expressed as ○: non-sticking, X: sticking and △: good. According to the cracking of the layer of the tablet after tableting, the capping is expressed as ○: no capping and X: occurrence of capping. The mold release and sticking were evaluated by visual observation, and the degree of capping was evaluated by separating the tablet by applying a physical force to the tablet by hand.

**[Table 1]**

| | Manufacturing Process | Raw material | Example 1 | Example 2 | Example 3 | Example 4 |
|---|---|---|---|---|---|---|
| 1 | Granulation | CG200745PPA | 125 | 125 | 125 | 125 |
| 2 | | Mannitol | 200 | 200 | 200 | 200 |
| 3 | | Microcrystalline cellulose | 200 | 200 | 200 | 200 |
| 4 | | Silicon dioxide | 5 | 5 | 5 | 5 |
| 6 | | Magnesium aluminometasilicate | 10 | 10 | 10 | 10 |
| 7 | | Hydroxypropyl cellulose | 15 | 15 | 15 | 15 |
| 8 | Post mixing | Glyceryl behenate | 10 | 10 | 30 | 50 |
| 9 | | Talc | 10 | - | 10 | 10 |
| 10 | | Sodium stearyl fumarate | - | 10 | - | - |
| Total weight | | | 575 | 575 | 595 | 615 |

**[Table 2]**

| Evaluation of tableting properties | Example 1 | Example 2 | Example 3 | Example 4 |
|---|---|---|---|---|
| Sticking | ○ | ○ | ○ | ○ |
| Mold release | Poor | Poor | Poor | Poor |
| Capping | X | X | X | X |

As a result of Examples 1 to 4, it was found that even when the ratio of the main ingredients to the excipient was 2:8, the tablet was jammed in the punch, making it difficult to be ejected, and a sticking phenomenon occurred in which the mixture adhered to the punch surface. These were not significantly improved even by changing the type of the lubricant or increasing the amount of the lubricant.

### Examples 5 to 8

In order to improve the sticking phenomenon, tablets were manufactured by increasing the amount of excipients and decreasing the portion of the main ingredients as shown in Table 3. In addition, Table 4 shows the results of tableting evaluation.

**[Table 3]**

| | Manufacturing Process | Raw material | Example 5 | Example 6 | Example 7 | Example 8 |
|---|---|---|---|---|---|---|
| 1 | Granulation | CG200745PPA | 125 | 125 | 125 | 125 |
| 2 | | Mannitol | 220 | 250 | 250 | 280 |
| 3 | | Microcrystalline cellulose | 200 | 220 | 220 | 220 |
| 4 | | Silicon dioxide | 5 | 5 | 5 | 5 |
| 7 | | Hydroxypropyl cellulose | 30 | 30 | 30 | 30 |
| 8 | Post mixing | Glyceryl behenate | 30 | 30 | 50 | 50 |
| 9 | | Talc | 10 | 10 | 10 | 10 |
| | Total weight | | 620 | 670 | 690 | 720 |

**[Table 4]**

| Evaluation of tableting properties | Example 5 | Example 6 | Example 7 | Example 8 |
|---|---|---|---|---|
| Sticking | ○ | ○ | X | X |
| Mold release | Poor | Poor | Good | Good |
| Capping | X | X | X | X |

### Examples 9 and 10

CG200745PPA can be applied as an anticancer agent. Most of the subjects to be administered are patients undergoing chemotherapy. Therefore, if increasing in tablet size, it is not only very difficult to take, but also there is a fear of rejection in taking tablets. Accordingly, in the present invention, it was intended to improve it. A capsule formulation composition was prepared that has less physical influence on the manufacture and a simple process such as a tableting process. As shown in the composition of Table 5, for convenience of dosing, the minimum amount of excipients that can be manufactured was used, and for simplification of the process, the direct mixing was used. In addition, the results of evaluating the flowability according to the angle of repose measurement criteria as shown in Table 6 (General Chapters; 1174, USP) using an angle of repose tester and the degree of sticking are shown in Table 7.

EMBO CAPS (hard capsule, manufactured by Suheung) was used for capsule #0 and capsule #1.

**[Table 5]**

| | Manufacturing Process | Raw material | Example 9 | Example 10 |
|---|---|---|---|---|
| 1 | Direct mixing | CG200745PPA | 125 | 125 |
| 2 | | Mannitol | 80 | 90 |
| 3 | | Microcrystalline cellulose | 70 | 80 |
| 6 | | Magnesium aluminometasilicate | 5 | 10 |
| 9 | | talc | 10 | 10 |
| 10 | | Sodium stearyl fumarate | 5 | 10 |
| Total weight | | | 295 | 325 |

**[Table 6]**

| Angles of Repose | |
|---|---|
| Flow Property | Angle of Repose |
| Excellent | 25-30 |
| Good | 31-35 |
| Fair-aid not needed | 36-40 |
| Passable-may hang up | 41-45 |
| Poor-must agitate, vibrate | 46-55 |
| Very poor | 56-65 |
| Very, very poor | >66 |

**[Table 7]**

| Evaluation of composition | Capsule # | Angle of repose | Sticking | Mass deviation |
|---|---|---|---|---|
| Example 9 | 1 | 46 | Δ | Mean 9% |
| Example 10 | 0 | 47 | X | Mean 7% |

### Examples 11 and 12

In order to improve flowability, a mixture was prepared in the form of granules, and a composition capable of filling in capsule #1 was prepared according to Table 8. In addition, the flowability, degree of sticking and mass deviation are shown in Table 9.

**[Table 8]**

| | Manufacturing Process | Raw material | Example 11 | Example 12 |
|---|---|---|---|---|
| 1 | Granulation | CG200745PPA | 125 | 125 |
| 2 | | Mannitol | 75 | 60 |
| 3 | | Microcrystalline cellulose | 70 | 60 |
| 6 | | Hydroxypropyl cellulose | 10 | 8 |
| 9 | | Talc | 10 | 7 |
| Total weight | | | 290 | 260 |

**[Table 9]**

| Evaluation of composition | Capsule # | Angle of repose | Sticking | Mass deviation |
|---|---|---|---|---|
| Example 11 | 1 | 29 | X | 3% or less |
| Example 12 | 1 | 31 | X | 3% or less |

The capsules filled with granules showed good flowability and little mass deviation. A total weight of 290 mg was considered to be problematic when filling into Capsule #1 due to the large volume characteristics of the mixture. Accordingly, a composition with a total weight of mixture of 260 mg was selected.

### Experimental Example 1: Evaluation of dissolution of tablet

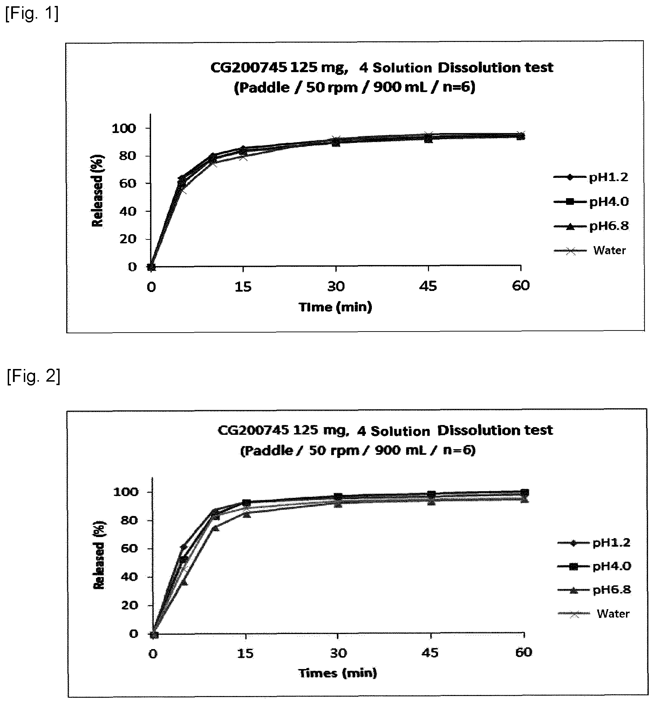

In accordance with Korean Pharmacopoeia dissolution test method 2 (paddle method, apparatus 2), the formulation of Example 8 was subjected to an in vitro dissolution test. The dissolution tests were carried out in all 4 solutions, and the results are shown in Fig. 1 and Table 10.

**[Table 10]**

| Solution | Min | 0 | 5 | 10 | 15 | 30 | 45 | 60 |
|---|---|---|---|---|---|---|---|---|
| pH 1.2 | Mean | 0 | 64.3 | 80.4 | 85.5 | 90.9 | 92.9 | 94.1 |
| | SD | 0 | 3.1 | 3.3 | 3 | 2.3 | 2 | 1.8 |
| pH 4.0 | Mean | 0 | 59.7 | 77.8 | 83.3 | 89.3 | 91.8 | 93 |
| | SD | 0 | 3.7 | 2.4 | 2.1 | 1.2 | 1.1 | 0.8 |
| pH 6.8 | Mean | 0 | 63.3 | 77.6 | 83.4 | 89 | 91.5 | 93.7 |
| | SD | 0 | 4.3 | 5 | 2.1 | 2 | 1.2 | 1.2 |
| water | Mean | 0 | 55.7 | 74.8 | 79.1 | 91.8 | 94.6 | 94.8 |
| | SD | 0 | 4.8 | 3.3 | 2.5 | 1.7 | 1.3 | 1.5 |

### Experimental Example 2: Evaluation of dissolution of capsule

In the same manner as in Experimental Example 1, in accordance with Korean Pharmacopoeia dissolution test method 2 (paddle method, apparatus 2), the formulation of Example 12 was subjected to an in vitro dissolution test. The dissolution tests were carried out in all 4 solutions, and the results are shown in Fig. 2 and Table 11.

**[Table 11]**

| Solution | min | 0 | 5 | 10 | 15 | 30 | 45 | 60 |
|---|---|---|---|---|---|---|---|---|
| pH 1.2 | Mean | 0 | 61.7 | 86.7 | 92.4 | 95.3 | 96.2 | 97 |
| | SD | 0 | 13.6 | 9.1 | 4.7 | 4 | 3.1 | 3.3 |
| pH 4.0 | Mean | 0 | 53 | 83.5 | 92.6 | 96.9 | 98.3 | 99.2 |
| | SD | 0 | 7.7 | 8.4 | 4.6 | 3.3 | 2.3 | 2.1 |
| pH 6.8 | Mean | 0 | 37.1 | 74.8 | 84.8 | 91.7 | 93.2 | 94 |
| | SD | 0 | 6.1 | 6.6 | 2.3 | 1.9 | 1.7 | 1.3 |
| water | Mean | 0 | 46.2 | 82.8 | 88.7 | 93.1 | 94.1 | 94.9 |
| | SD | 0 | 7.4 | 6 | 3.8 | 2.7 | 2.1 | 1.7 |

### Experimental Example 3: Evaluation of related substances of capsule composition of Example 12

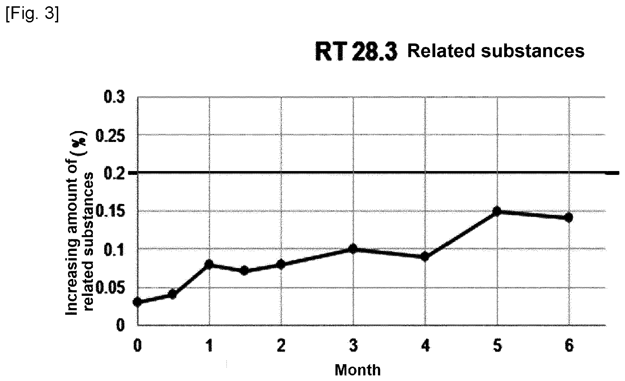

In order to evaluate the amount of change in the related substances of Example 12, the composition of Example 12 was placed in an accelerated condition chamber (45°C±2, 75%±5), and the amount of related substance produced was measured for 6 months. For the measurement of the amount of related substances produced, the composition of Example 12 was pretreated and then tested according to the liquid chromatography method of the general test method of the Korean Pharmacopoeia to determine peak areas of the produced related substances (individual related substance 0.2% or less, total related substances 1.0%, measuring equipment: HPLC LC-2030C_Shimadzu). The results are shown in Table 12 and Fig. 3. In Table 12, the amount of related substances produced is expressed in %. In addition, Fig. 3 is a result of measuring the related substance at 28 minutes under the accelerated condition, and the reference value is 0.2%.

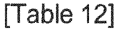

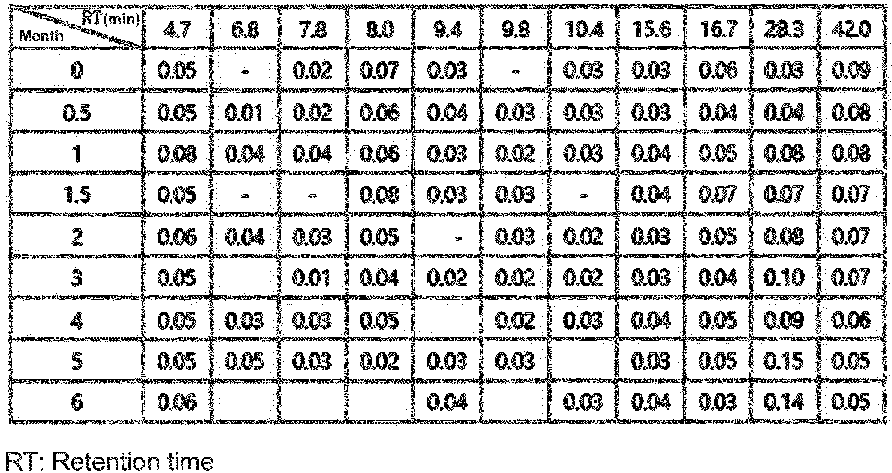

As shown in Table 12 and Fig. 3, the amounts of other related substances produced except for the peak of RT 28.3 mins were insignificant. Further, it was confirmed that the amount of the related substance for the peak of RT 28.3 meets the criteria for individual related substance for 6 months under the accelerated condition.

### Experimental Example 4: Evaluation of stability

In order to evaluate the stability, an alkylcarbamoyl naphthalenyloxy octenoyl hydroxyamide compound was used in Comparative Example 1, an alkyl carbamoyl naphthalenyloxy octenoyl hydroxyamide tartrate was used in Example 13 and alkylcarbamoyl naphthalenyloxy octenoyl hydroxyamide phosphate was used in Example 14. As alkylcarbamoyl naphthalenyloxy octenoyl hydroxyamide, (E)-N1-(3-(dimethylamino)propyl)-N8-hydroxy-2-((naphthalen-1-yloxy)methyl)-2-octenediamide compound was used. Evaluation of stability was conducted by observing the change with storage time under room temperature (20-25°C, 50% or less), long-term (25±2°C and 60±5%RH), accelerated (40±2°C and 75±5%RH) and severe (60±2°C) conditions.

### Experimental Example 4-1: Evaluation of change in moisture content

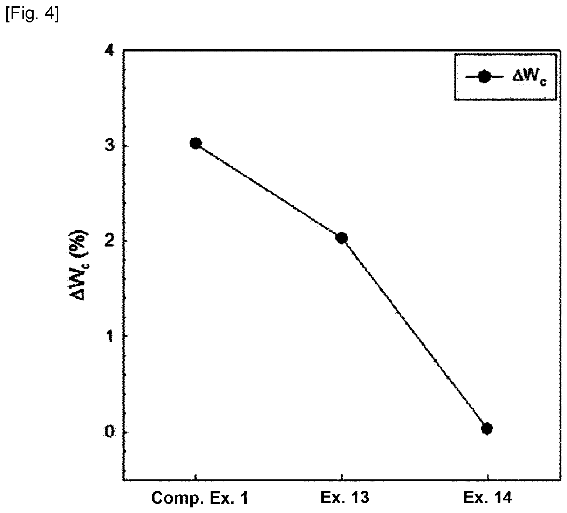

In order to evaluate the change in moisture content, the compounds according to Examples and Comparative Examples were stored in an open state at room temperature (20 to 25°C, 50% or less), and the change was measured by the difference between the initial moisture content and the moisture content after 3 days, and the results are shown in Fig. 4. Moisture content was measured according to the volumetric titration method among methods of determining the water content (Karl Fischer method) of the general test methods of the Korean Pharmacopoeia (measurement equipment: 701KF_Metrohm).

As shown in Fig. 4, in the case of Comparative Example 1, the moisture content was increased by about 3%, in the case of Example 13, the moisture content was increased by 2%, and in the case of Example 14, the change in the moisture content was very slight as about 0.01%.

### Experimental Example 4-2: Evaluation of change in appearance

In order to evaluate the change in appearance of the compounds according to Comparative Examples and Examples, each compound was stored in an open state according to the conditions shown in Table 13 below, and then the change in appearance was observed.

**[Table 13]**

| Example | Storage period | Room temperature | Long-term | Accelerated | Severe |
|---|---|---|---|---|---|
| Comp. Ex. 1 | Initial | Foam | | | |
| | 1 day | Gel-like | Gel-like | Gel-like | Gel-like |
| | 7 days | - | - | - | - |
| Ex. 13 | Initial | Powder | | | |
| | 1 day | Powder | Gel-like | Gel-like | Powder |
| | 7 days | Powder (white) | - | - | Powder Discoloration (pale yellow) (Particle size reduction) |
| Ex. 14 | Initial | Powder | | | |
| | 1 day | Powder | Powder | Powder | Powder |
| | 7 days | Powder | Powder | Powder | Powder |

As shown in Table 13, in the case of Comparative Example 1, as moisture was absorbed, the appearance was changed from a foam to a highly viscous liquid or gel after 1 day (24 hours) elapsed, and in the case of Example 14, it can be found that the appearance remained constant regardless of the condition.

### Experimental Example 4-3: Evaluation of change in content

In order to evaluate the change in the content of the compounds according to Examples, each compound was stored in an open state or in a polyethylene bottle packaging (silica gel added) to measure the change in content. For measurement of content, compounds of Comparative Examples and Examples were pretreated and then the solutions were tested according to the liquid chromatography method of the general test methods of the Korean Pharmacopoeia to determine the peak area of CG200745 (measurement equipment: HPLC LC-2030C_Shimadzu). The content standard is to contain CG200745 in an amount corresponding to 95.0-105.5% of the indicated amount according to the standards and test methods of the present applicant.

The results measured in the open state are shown in Table 14 below, and the results measured in the bottle packaging are shown in Table 15 below.

**[Table 14]**

| Example | Storage period | Room temperature | Long-term | Accelerated | Severe |
|---|---|---|---|---|---|
| Comp. Ex. 1 | Initial | 98.9 | | | |
| | 1 day | 101.0 | 99.9 | 92.3 | 101.1 |
| | 3 days | 101.9 | N.T. | N.T. | 97.2 |
| | 7 days | 100.1 | N.T. | N.T. | 89.1 |
| Ex. 13 | Initial | 99.5 | | | |
| | 1 day | 103.4 | 104.3 | 104.6 | 103.3 |
| | 3 days | 102.6 | 102.6 | 102.3 | 99.6 |
| | 7 days | 100.0 | 99.4 | 100.7 | 102.2 |

| | | | | | |
|---|---|---|---|---|---|
| N.T: As the change in appearance was observed, the test was not performed. | | | | | |

**[Table 15]**

| Example | Storage period | Room temperature | Long-term | Accelerated | Severe |
|---|---|---|---|---|---|
| Comp. Ex. 1 | Initial | 98.9 | | | |
| | 1 day | 99.3 | 99.1 | 89.1 | 101.2 |
| | 3 days | 99.5 | 84.0 | 84.0 | N.T. |
| | 7 days | 99.4 | 78.5 | 78.5 | N.T. |
| Ex. 14 | Initial | 99.5 | | | |
| | 1 day | 98.6 | 103.5 | 101.2 | 101.6 |
| | 3 days | 98.8 | 99.9 | 100.1 | 100.4 |
| | 7 days | 99.3 | 100.9 | 101.1 | 101.4 |

| | | | | | |
|---|---|---|---|---|---|
| N.T: As the change in appearance was observed, the test was not performed. | | | | | |

Summarizing the results of Experimental Example 4, it was found that in the case of Example 13, the content was maintained at a certain level under the room temperature condition, and in the case of Example 14, the content was kept constant regardless of packaging conditions, heating, and humidity.

### Experimental Example 5: Comparison of the amount of change in related substances

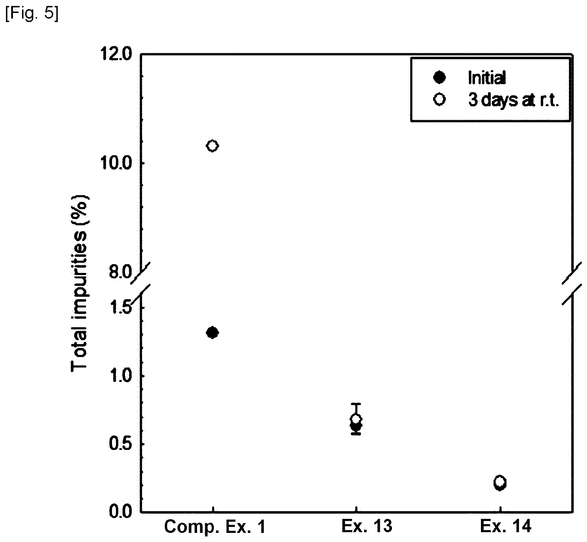

In order to evaluate the amount of change in related substances of the compounds according to Comparative Examples and Examples, each compound was stored in an open state at room temperature for 3 days, and then the amount of related substances produced was measured in the same manner as in Experimental Example 3 and the results are shown in Fig. 5. As shown in Fig. 5, the amount of related substances was increased by about 10% in Comparative Example 1, while the amount was increased by 0.05% in Example 13 and the amount was increased by 0.02% in Example 14, which were relatively insignificant.

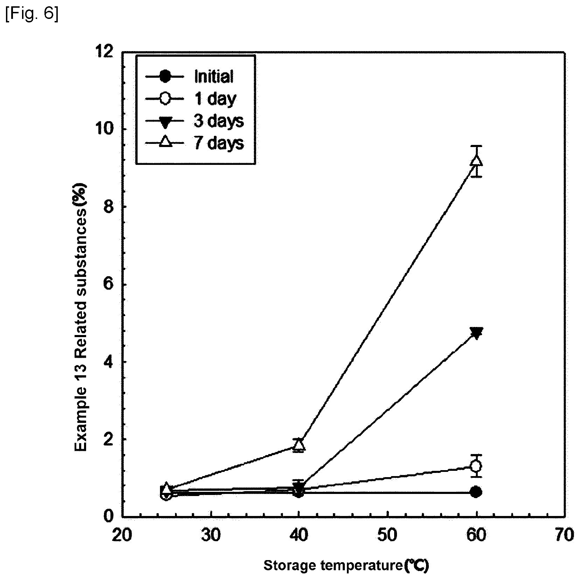

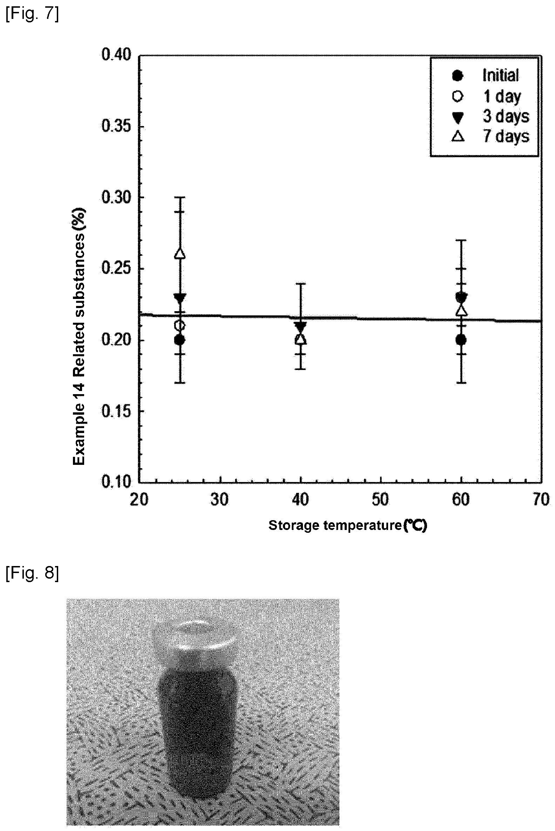

In addition, changes in the amount of related substances produced according to temperature and storage period are shown in Figs. 6 and 7 for the compounds according to Examples 13 and 14. As shown in Fig. 6, the amount of related substances produced in Example 13 was maintained within a certain range at a relatively low temperature. As shown in Fig. 7, the amount of related substances produced in Example 14 was kept constant within a certain range of about 0.22% regardless of the storage temperature and period.

### Example 15

Since CG200745PPA is an anticancer drug that can be administered to cancer patients for the purpose of cancer treatment, various routes of administration should be considered according to the patient's condition. Thus, the route that can be administered to a subject having difficulties in oral administration includes intravenous or intramuscular injection. For this, there are injections in a liquid form or a lyophilized form that is diluted immediately before use. The composition as shown in Table 16 without using a solubilizing agent and other additives for improving stability in the dissolved state and compatibility with other additives, was dissolved in water for injection purged with nitrogen, and lyophilized. A photograph of the prepared composition is shown in Fig. 8.

**[Table 16]**

| | Raw material | Example 15 |
|---|---|---|
| 1 | CG200745PPA | 125 |
| 2 | Water for injection (specific gravity 1.0 g/mL) | To 5 mL |
| 3 | Nitrogen | q.s. |

As can be seen from the above results, the CG200745PPA compound can improve the dissolution rate while maintaining properties such as medicinal efficacy and effective amount.

Therefore, it can be easily applied to a subject to be administered as an anticancer agent related to HDAC inhibition, for example, oral administration and injection.

## Claims

1. A pharmaceutical composition comprising a compound of formula 1, a compound of formula 2 or a combination thereof in the form of a tablet, granule, powder, capsule, dry syrup or formulated for injection: 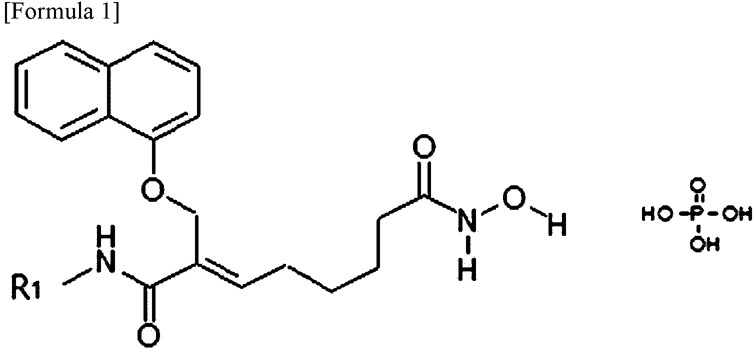 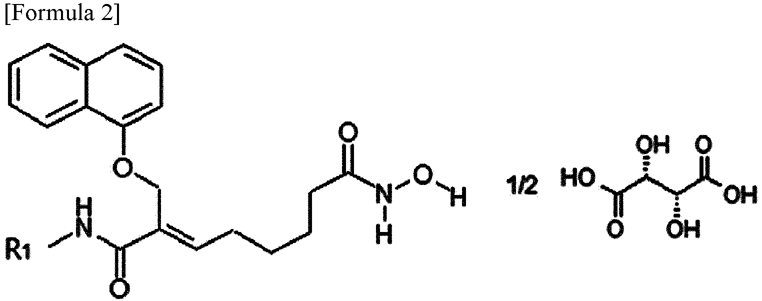 wherein in Formula 1 or 2,
R₁ is -CH₂CH₂CH₂N(CH₃)₂.

2. The pharmaceutical composition according to claim 1, wherein the pharmaceutical composition is a tablet or capsule, wherein the tablet or capsule further comprises a coating layer, and the coating layer is present in an amount of 1 to 10% by weight based on weight of the tablet or capsule.

3. The pharmaceutical composition according to claim 1, wherein the pharmaceutical composition formulated for injection is in a liquid or a lyophilized form.

4. A pharmaceutical composition for use in treating cancer, comprising the compound of formula 1, the compound of formula 2, or a combination thereof according to claim 1.

5. A method for preparing a pharmaceutical composition comprising a compound of formula 1, a compound of formula 2 or a combination thereof, the method comprising:
1)forming a free base solution of (E)-N1-(3-(dimethylamino)propyl)-N8-hydroxy-2-((naphthalen-1-yloxy)methyl)-2-octenediamide in a first organic solvent, and
2) adding phosphoric acid or tartaric acid to the free base solution;
wherein the pharmaceutical composition is in the form of a tablet, granule, powder, capsule, dry syrup or formulated injection: 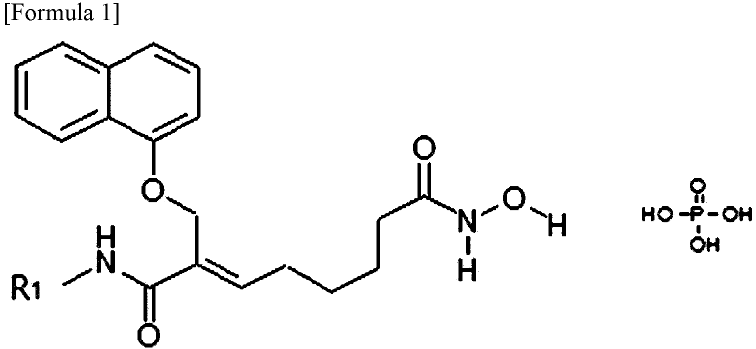 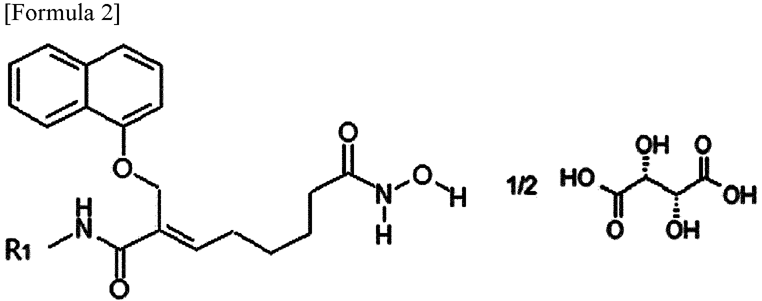
wherein in the Formula 1 or 2,
R₁ is -CH₂CH₂CH₂N(CH₃)₂.

6. The method according to claim 5, further comprising additionally adding a second solvent having a lower solubility than the first organic solvent of step 1).

7. The method according to claim 5 or 6, wherein the first organic solvent of step 1) comprises one or more solvents selected from the group consisting of methanol, ethanol, propanol, tetrahydrofuran, chloroform, N,N-dimethylformamide (DMF), dimethyl sulfoxide (DMSO), acetonitrile and ethyl acetate.

8. The method according to claim 6, wherein the second solvent having a lower solubility than the first organic solvent of step 1) comprises one or more solvents selected from the group consisting of alcohols including methanol, ethanol and propanol, tetrahydrofuran, acetonitrile and acetone.

9. The method according to any one of claims 5 to 8, wherein the method further comprises sterilizing the pharmaceutical composition.

10. The method according to claim 9, wherein the sterilizing is achieved by a high-temperature decompression sterilization method or aseptic filtration method.

## Patentansprüche

1. Pharmazeutische Zusammensetzung, die eine Verbindung der Formel 1, eine Verbindung der Formel 2 oder eine Kombination hiervon in Form einer Tablette, eines Granulats, eines Pulvers, einer Kapsel, eines Trockensirups oder einer Formulierung für eine Injektion umfasst: 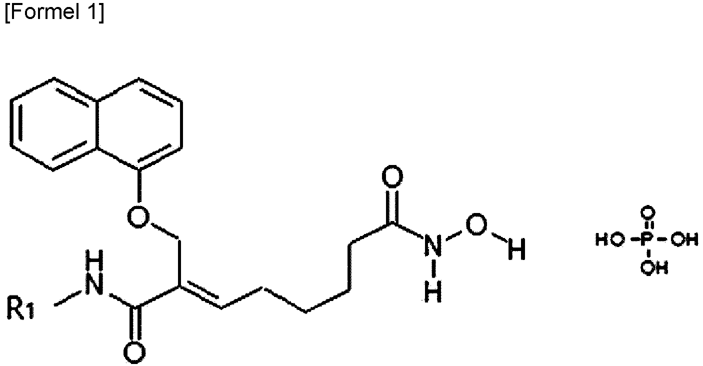 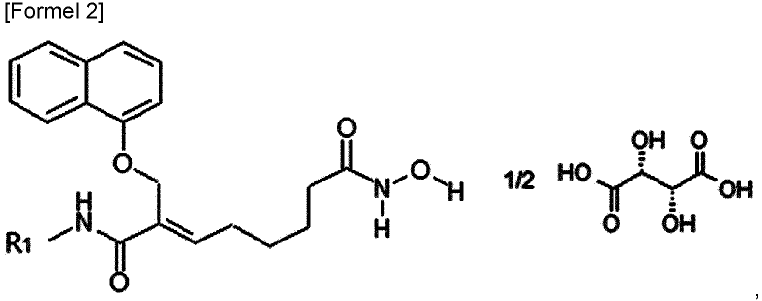 wobei in Formel 1 oder in Formel 2 R₁ für -CH₂CH₂CH₂N(CH₃)₂ steht.

2. Pharmazeutische Zusammensetzung gemäß Anspruch 1, wobei die pharmazeutische Zusammensetzung eine Tablette oder eine Kapsel ist, wobei die Tablette oder die Kapsel des Weiteren eine Überzugsschicht umfasst und die Überzugsschicht in einer Menge von 1 bis 10 Gew.-%, bezogen auf das Gewicht der Tablette oder der Kapsel, vorhanden ist.

3. Pharmazeutische Zusammensetzung gemäß Anspruch 1, wobei die für eine Injektion formulierte pharmazeutische Zusammensetzung in flüssiger oder in lyophilisierter Form vorliegt.

4. Pharmazeutische Zusammensetzung zur Verwendung bei der Behandlung von Krebs, wobei die pharmazeutische Zusammensetzung die Verbindung der Formel 1, die Verbindung der Formel 2 oder eine Kombination hiervon gemäß Anspruch 1 umfasst.

5. Verfahren zur Herstellung einer pharmazeutischen Zusammensetzung, die eine Verbindung der Formel 1, eine Verbindung der Formel 2 oder eine Kombination hiervon umfasst, wobei das Verfahren Folgendes umfasst:
1)Bilden einer Lösung der freien Base von (E)-N1-(3-(Dimethylamino)propyl)-N8-hydroxy-2-((naphthalin-1-yloxy)methyl)-2-octendiamid in einem ersten organischen Lösemittel und
2) Hinzufügen von Phosphorsäure oder Weinsäure zu der Lösung der freien Base,
wobei die pharmazeutische Zusammensetzung in Form einer Tablette, eines Granulats, eines Pulvers, einer Kapsel, eines Trockensirups oder einer formulierten Injektion vorliegt: 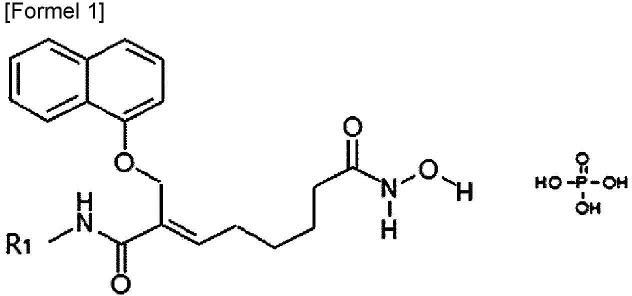 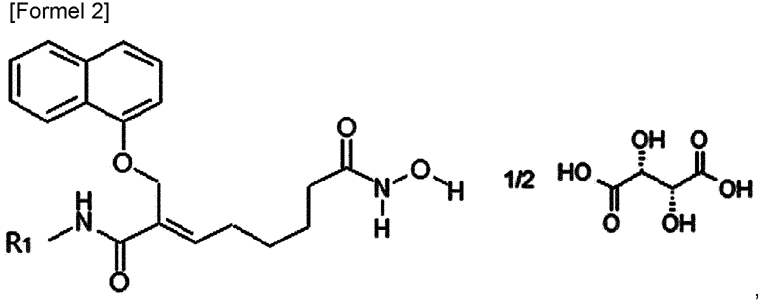
wobei in Formel 1 oder in Formel 2 R₁ für -CH₂CH₂CH₂N(CH₃)₂ steht.

6. Verfahren gemäß Anspruch 5, das des Weiteren das zusätzliche Zugeben eines zweiten Lösemittels, das eine geringere Löslichkeit als das erste organische Lösemittel von Schritt 1) aufweist, umfasst.

7. Verfahren gemäß Anspruch 5 oder Anspruch 6, wobei das erste organische Lösemittel von Schritt 1) ein oder mehrere Lösemittel umfasst, die aus der Gruppe ausgewählt sind, die aus Methanol, Ethanol, Propanol, Tetrahydrofuran, Chloroform, N,N-Dimethylformamid (DMF), Dimethylsulfoxid (DMSO), Acetonitril und Ethylacetat besteht.

8. Verfahren gemäß Anspruch 6, wobei das zweite Lösemittel, das eine geringere Löslichkeit als das erste organische Lösemittel von Schritt 1) aufweist, ein oder mehrere Lösemittel umfasst, die aus der Gruppe ausgewählt sind, die aus Alkoholen, einschließlich Methanol, Ethanol und Propanol, Tetrahydrofuran, Acetonitril und Aceton besteht.

9. Verfahren gemäß einem der Ansprüche 5 bis 8, wobei das Verfahren des Weiteren ein Sterilisieren der pharmazeutischen Zusammensetzung umfasst.

10. Verfahren gemäß Anspruch 9, wobei das Sterilisieren mittels eines Hochtemperatur-Dekompressions-Sterilisationsverfahrens oder eines aseptischen Filtrationsverfahrens erreicht wird.

## Revendications

1. Composition pharmaceutique comprenant un composé de formule 1, un composé de formule 2 ou une de leurs combinaisons sous la forme d'un comprimé, d'un granulé, d'une poudre, d'une capsule, d'un sirop anhydre ou formulée pour injection 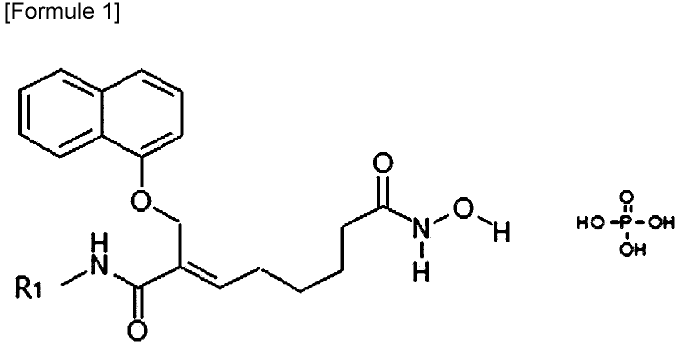 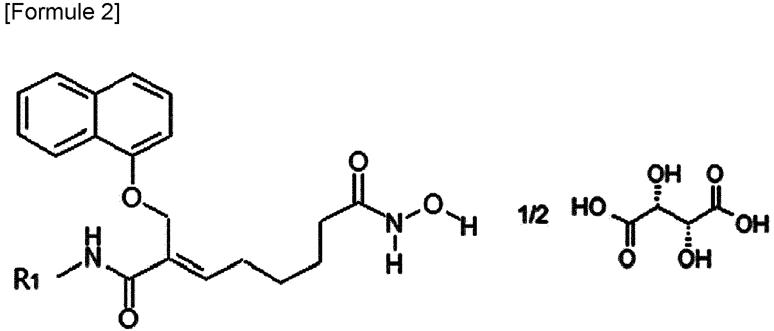 dans laquelle dans la formule 1 ou 2, R₁ est un groupe -CH₂CH₂CH₂N(CH₃)₂.

2. Composition pharmaceutique selon la revendication 1, dans laquelle la composition pharmaceutique est un comprimé ou une capsule, le comprimé ou la capsule comprenant en outre une couche de revêtement et la couche de revêtement est présente en une quantité de 1 à 10 % en poids sur la base du poids du comprimé ou de la capsule.

3. Composition pharmaceutique selon la revendication 1, dans laquelle la composition pharmaceutique formulée pour injection est dans une forme liquide ou une forme lyophilisée.

4. Composition pharmaceutique destinée à être utilisée dans le traitement du cancer, comprenant le composé de formule 1, le composé de formule 2 ou une de leurs combinaisons selon la revendication 1.

5. Procédé de préparation d'une composition pharmaceutique comprenant un composé de formule 1, un composé de formule 2 ou une de leurs combinaisons, le procédé comprenant les étapes consistant à :
1) former une solution de base libre de (E)-N1-(3-(diméthylamino)propyl)-N8-hydroxy-2-((naphtalène-1-yloxy)méthyl)-2-octènediamide dans un premier solvant organique et
2) ajouter de l'acide phosphorique ou de l'acide tartrique à la solution de base libre ;
la composition pharmaceutique étant sous la forme d'un comprimé, d'un granulé, d'une poudre, d'une capsule, d'un sirop anhydre ou formulée pour injection : 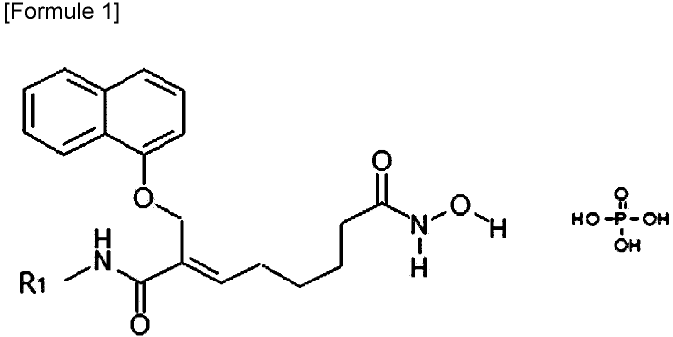 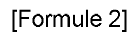 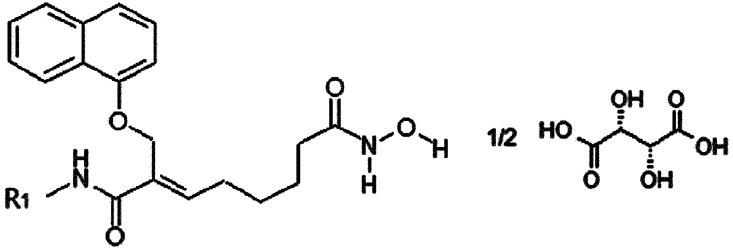
dans laquelle dans la formule 1 ou 2, R₁ est un groupe -CH₂CH₂CH₂N(CH₃)₂.

6. Procédé selon la revendication 5, comprenant en outre de plus l'addition d'un second solvant ayant une solubilité inférieure au premier solvant organique de l'étape 1).

7. Procédé selon la revendication 5 ou 6, dans lequel le premier solvant organique de l'étape 1) comprend un ou plusieurs solvants choisis dans le groupe constitué par le méthanol, l'éthanol, le propanol, le tétrahydrofurane, le chloroforme, le N,N-diméthylformamide (DMF), le diméthyl sulfoxyde (DMSO), l'acétonitrile et l'acétate d'éthyle.

8. Procédé selon la revendication 6, dans lequel le second solvant ayant une solubilité inférieure au premier solvant organique de l'étape 1) comprend un ou plusieurs solvants choisis dans le groupe constitué par les alcools comprenant le méthanol, l'éthanol et le propanol, le tétrahydrofurane, l'acétonitrile et l'acétone.

9. Procédé selon l'une quelconque des revendications 5 à 8, dans lequel le procédé comprend en outre la stérilisation de la composition pharmaceutique.

10. Procédé selon la revendication 9, dans lequel la stérilisation est obtenue par un procédé de stérilisation par décompression à haute température ou un procédé de filtration aseptique.
